Europäisches Patentamt
European Patent Office
Office européen des brevets

(19)

(11) Veröffentlichungsnummer: **0 005 785**
**A1**

(12)

# EUROPÄISCHE PATENTANMELDUNG

(21) Anmeldenummer: 79101549.8

(22) Anmeldetag: 22.05.79

(51) Int. Cl.[2]: C 07 D 501/00
A 61 K 31/545
//C07D277/40, C07D277/42

(30) Priorität: 26.05.78 DE 2822861

(43) Veröffentlichungstag der Anmeldung: 12.12.79 Patentblatt 79/25

(84) Benannte Vertragsstaaten: DE FR GB

(71) Anmelder: HOECHST AKTIENGESELLSCHAFT
Zentrale Patentabteilung Postfach 80 03 20
D-6230 Frankfurt/Main 80(DE)

(72) Erfinder: Bormann, Dieter, Dr.
Johann-Strauss-Strasse 20
D-6233 Kelkheim (Taunus)(DE)

(72) Erfinder: Dürckheimer, Walter, Dr.
Im Lerchenfeld 45
D-6234 Hattersheim(DE)

(72) Erfinder: Schrinner, Elmar, Dr.
Hedwigstrasse 2
D-6200 Wiesbaden(DE)

(54) **Cephemderivate, Verfahren zu ihrer Herstellung, pharmazeutische Präparate auf Basis dieser Verbindungen und ihre Verwendung.**

(57) Cephemderivate der allgemeinen Formel

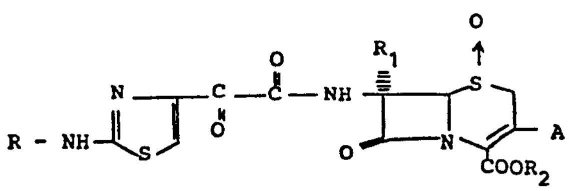

Verfahren zu ihrer Herstellung und gegen bakterielle Infektionen wirksame pharmazeutische Präparate, die solche Cephemderivate enthalten.

EP 0 005 785 A1

Croydon Printing Company Ltd.

HOECHST AKTIENGESELLSCHAFT HOE 78/F 100 . Dr.KA/E

**Cephemderivate, Verfahren zu ihrer Herstellung, pharmazeutische Präparate auf Basis dieser Verbindungen und ihre Verwendung**

Gegenstand der Erfindung sind Cephemderivate der allgemeinen Formel

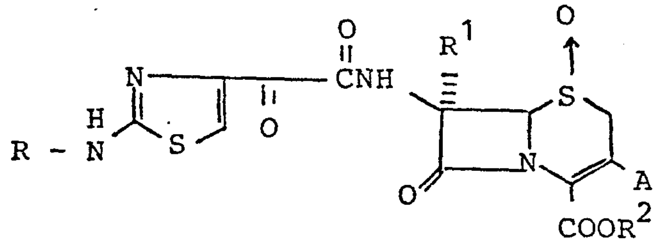

I

in der R ein Wasserstoff oder eine Aminoschutzgruppe, $R^1$ Wasserstoff oder niedrigmolekulares Alkoxy, $R^2$ Wasserstoff oder ein pharmazeutisch unbedenkliches Kation und A eine Methylgruppe, eine Gruppe $-CH_2O$-Acyl mit niedrigmolekularem Acyl

oder einen Rest $-CH_2-SR^3$ darstellen kann, in dem $R^3$ für einen gegebenenfalls substituierten 5- oder 6-gliedrigen heterocyclischen Ring mit 1 bis 4 Heteroatomen steht.

Gegenstand der Erfindung ist weiterhin das Verfahren zur Herstellung von Verbindungen der allgemeinen Formel I, das dadurch gekennzeichnet ist, daß man Cephemverbindungen der allgemeinen Formel

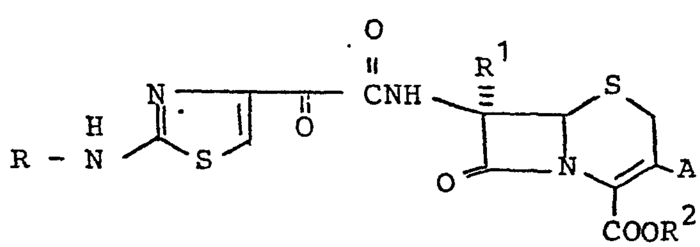

II

in der die Reste R, $R^1$, $R^2$ und A die oben angegebenen Bedeutungen haben, oxydiert und, falls gewünscht, in Verbindungen der Formel I den Rest R in der Bedeutung einer Aminoschutzgruppe in an sich bekannter Weise entfernt.

In den allgemeinen Formeln I und II steht R für Wasserstoff oder eine Aminoschutzgruppe. Als Schutzgruppen dienen die in der Peptidchemie üblicherweise verwendeten Gruppierungen, wie z.B. Formyl, Halogenacetyl, insbesondere Brom- oder Chloracetyl, oder t-Butoxycarbonyl. Insbesondere wird die Triphenylmethylgruppe verwendet.

Steht $R^1$ für einen niedrigmolekularen Alkoxyrest, so kommen Reste mit 1 bis 4 C-Atomen, wie z.B. Methoxy, Äthoxy, vorzugsweise Methoxy in Betracht.

Steht A für den Rest $-CH_2-S-R^3$, so kann $R^3$ die Bedeutung eines 5- oder 6-gliedrigen heterocyclischen Ringes mit 1 bis 4 Heteroatomen besitzen, der auch noch substituiert sein kann. Als Heteroatome kommen insbesondere Sauerstoff, Schwefel und/oder Stickstoff in Betracht. Als Beispiele für derartige Ringsysteme seien genannt. Imidazolyl, Pyrazolyl, Thiazolyl, Isothiazolyl, Oxazolyl, Isoxazolyl, Triazolyl,Thiadiazolyl, Oxdiazolyl, Tetrazolyl, Pyridyl, Pyrimidyl, Pyrazinyl, Pyridazinyl, Thiazinyl. Bevorzugt sind 5-gliedrige Ringe, die 1 bis 4 Stickstoffatome und gegebenenfalls ein Schwefelatom enthalten, wie z.B. Thiazol-2-yl, 1,3,4-Thiadiazol-2-yl, 4-H-1,2,4-Triazol-3-yl, 1-H-Tetrazol-5-yl.

Bedeutet A eine Gruppe $-CH_2O$-Acyl, so kommen als "Acyl" niedrigmolekulare Acylreste mit 1 bis 5 C-Atomen in Betracht, wie z.B. die Formyl-, Acetyl- oder Propionylgruppe, insbesondere aber die Acetylgruppe. Als Substituenten der heterocyclischen Ringe kommen die aus der Cephalosporinchemie für derartige Ringe bekannten Substituenten in Betracht, insbesondere Alkylgruppen mit 1 bis 5 C-Atomen,

wie beispielsweise Methyl, Äthyl, Propyl, Isopropyl, Allyl oder n-Pentyl, vorzugsweise Methyl und Äthyl oder die Aminogruppe.

Steht $R^2$ für ein pharmazeutisch unbedenkliches Kation, so kommen als solche anorganische und organische Kationen in Betracht. Bei den anorganischen sind die Alkali- und Erdalkalisalze bevorzugt, insbesondere das Natrium-, Magnesium- und Calciumsalz. Als organische Kationen kommen vor allem die stickstoffhaltigen Kationen in Betracht, wie z.B. Trialkylammonium mit niedrigmolekularem Alkyl, insbesondere Triäthylammonium, aber auch das Procain.

Die für die erfindungsgemäße Reaktion einzusetzenden Cephemverbindungen der Formel II sind bekannt. Es ist weiterhin bekannt, daß sich Cephemverbindungen am Schwefel oxydieren lassen, wobei je nach Wahl des Oxydationsmittels α- oder ß-S-Oxyde entstehen (E. Flynn, Cephalosporins and Penicillins, Chemistry and Biology, Academic Press, 1972, S. 135 ff.). Es ist darüberhinaus bekannt, daß einige α-S-Oxyde eine antibakterielle Wirkung besitzen, während die ß-S-Oxyde antibiotisch unwirksam sind (vgl. E. Flynn, S. 577).

Es wurde nun überraschenderweise gefunden, daß sich die 7-(2-NHR-thiazol-4-yl-glyoxylamido)-cephemverbindungen der Formel II glatt und in hohen Ausbeuten zu den 7-(2-NHR-thiazol-4-yl-glyoxylamido)-cephem-ß-S-oxyd-carbonsäuren oxydieren lassen und daß die erhaltenen Cephem-ß-S-oxyde unerwartete antibakterielle Aktivität besitzen.

Die Oxydation läßt sich auf verschiedene Weise durchführen. Eine bevorzugte Methode besteht darin, eine Substanz der allgemeinen Formel II in Eisessig oder in wäßrigem Eisessig vorzulegen und die Reaktionsmischung mit Persäuren zu oxydieren. Als Persäuren eignen sich z.B. m-Chlorperbenzoesäure oder Peressigsäure. Als Reaktionsprodukte bei dieser Verfahrensweise werden die ß-S-Oxyde erhalten. Die Persäuren werden in mindestens stöchiometrischer Menge eingesetzt. Ein geringer Überschuß kann von Vorteil sein. Die Reaktionstemperatur für die Oxydation ist nicht kritisch, jedoch empfiehlt es sich, sie zur Verhinderung von Sulfonbildungen in einem Temperaturbereich von -10 bis +20°C durchzuführen.

Den Reaktionsverlauf kann man am Abklingen der exothermen Reaktion verfolgen, besser aber noch im Dünnschichtchromatigramm am Verschwinden des Flecks für die Ausgangverbindung und der Entstehung eines neuen Fleckes mit einem niedrigeren Rf-Wert, z.B. im Fließmittel Butanol : Wasser : Äthanol : Essigsäure = 5 : 2 : 1,5 : 1,5

Die Isolierung der Endprodukte hängt von der Art der Substanz ab. Ist beispielsweise die Verbindung am Aminostickstoff trityliert, so kann man bei Verwendung eines mit Wasser mischbaren Lösungsmittels das entstandene Cephem-ß-S-oxyd durch Zusatz von Wasser ausfällen. Bei Verwendung von mit Wasser nicht mischbaren Lösungsmitteln, wie beispielsweise halogenierten Kohlenwasserstoffen, wie z.B. Methylenchlorid oder Chloroform, ist es ratsam, nach Zerstörung der überschüssigen Persäure, beispielsweise mit Eisen-II--Chlorid-Lösung, das Lösungsmittel zu entfernen und den Rückstand mit Wasser aufzunehmen.

Bei Verwendung der Verbindungen der Formel II, in der R für Wasserstoff steht, kann nach Zerstörung der Persäure das Lösungsmittel entfernt und der Rückstand mit organischen Lösungsmitteln kristallisiert, oder die ausgefallenen Kristalle oder Feststoffe direkt isoliert werden.

Hat man Verbindungen der Formel II oxydiert, in denen die Aminogruppe durch eine Schutzgruppe blockiert war, so läßt sich diese - falls gewünscht - nach literaturbekannten Verfahren entfernen. So kann man beispielsweise die

entsprechende Halogen-acetamido-Gruppierung durch Zusatz von Thioharnstoff in die Aminogruppe überführen oder die Triphenylmethylaminogruppe durch verdünnte Ameisensäure in Triphenylcarbinol und die Aminogruppe spalten.

Die Isolierung der Endprodukte der Formel I erfolgt in diesem Fall nach den üblichen Laboratoriumsmethoden wie sie oben beschrieben sind.

Hat man die Triphenylmethyl-Schutzgruppe in verdünnter Ameisensäure abgespalten, so empfiehlt es sich, das entstandene Triphenylcarbinol durch Extraktion zu entfernen.

Die Cephemderivate der Formel I sind wertvolle Antibiotica, die sich überraschend gut zur Bekämpfung grampositiver und vor allem gramnegativer Infekte eignen und darüber hinaus auch gegen penicillinasebildende Staphylokokken unerwartet gut wirksam sind.

Die Verbindungen der allgemeinen Formel I können auch dadurch erhalten werden, daß man 7-Aminocephalosporansäure-S-oxyde der allgemeinen Formel III

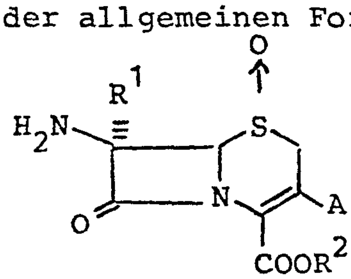

III

in der A, $R^1$ und $R^2$ die obengenannten Bedeutungen besiten, in an sich bekannter Weise mit zur Ambidbildung befähigten Derivaten einer Säure der allgemeinen Formel IV

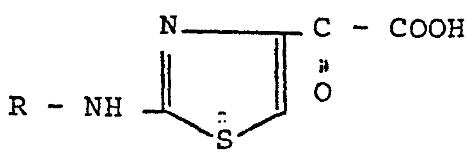

IV

in der R wie oben zu definieren ist, umsetzt. Als zur Amidbildung befähigte Derivate kommen beispielsweise symmetrische oder unsymmetrische Anhydride, insbesondere mit Pivaloylsäure, oder auch aktivierte Ester, insbesondere der 1-Hydroxybenztriazolester, in Betracht.

Die Herstellung der Verbindungen der Formel III kann aus den entsprechenden 7-Amino-cephalosporansäurederivaten durch Oxidation in der oben beschriebenen Weise erfolgen.

Die Ausgangsverbindungen der Formel IV sind literaturbeka-nt oder können nach literaturbekannten Methoden hergestellt werden.

Die erfindungsgemäßen Verbindungen können als solche oder zusammen mit den therapeutisch üblicherweise eingesetzten Hilfs- und Zusatzstoffen, wie z.B. Traganth, Milchzucker, Talkum, Lösungsmitteln, etc. in Form galenischer Zubereitungen, wie z.B. Tabletten, Dragées, Kapseln, Suspensionen, Lösungen etc. peroral, vorzugsweise jedoch parenteral eingesetzt werden, wobei der Wirkstoff in der Regel in einer Menge von etwa 50 bis 1000 mg, vorzugsweise etwa 100 bis 500 mg in einer Verabreichungseinheit enthalten ist.

Für die patenterale Anwendung kommen die für den therapeutischen Einsatz bekannten Lösungsmittel, insbesondere eine Lösung in Wasser in Betracht.

Es ist auch möglich, die erfindungsgemäßen Verbindungen mit anderen Wirkstoffen zu kombinieren. So können beispielsweise andere Antibiotica appliziert werden, wie z.B. solche aus der Reihe der Penicilline, Cephalosporine, oder Verbindungen, die die Symptomatik bakterieller Infektionen beeinflussen, wie z.B. Antipyretika, Antiphlogistica oder Analgetica.

Die folgenden Ausführungsbeispiele dienen zur weiteren Erläuterung der Erfindung, schränken sie jedoch nicht darauf ein.

Beispiel 1:

Eine Lösung von 5 g 7(2-(Triphenylmethylamino-thiazol-4-yl)-glyoxylamido)-cephalosporansäure in 40 ml 80 % Eisessig wird auf 0°C gekühlt, wobei eine Suspension entsteht, die anschließend mit einer Lösung von 4,0 g Peressigsäure (40 %) in 10 ml Eisessig unter Rühren so versetzt wird, daß die Temperatur nicht über 10°C steigt. Es bildet sich eine klare Lösung. Nach Ende der Zugabe wird noch 30 Min. gerührt, anschließend die Reaktionsmischung mit Wasser versetzt, wobei das entstandene 7(2-(Triphenylmethylamino-thiazol-4-al)-glyoxylamido)-cephalosporansäure-ß-S-oxyd in gelblichen Nadeln ausfällt. Das erhaltene Produkt wird isoliert, getrocknet und mit Äther verrieben und liefert das genannte S-Oxyd als blaßgelben Farbstoff vom Rf 0,57 (BuOH : $H_2O$ : Äthanol : Essigsäure = 5 : 2 : 1,5 : 1,5) IR im KBr : ß-Lactam 1795 $cm^{-1}$.

Beispiel 2:

In der im Beispiel 1 angegebenen Weise erhält man bei der Verwendung von 7(2-Triphenylmethylamino-thiazol-4-yl)-glyoxylamido)-3-[(5-methyl-1,3,4-thiadiazol-2-yl)-thiomethyl]-$\Delta$3-cephem-4-carbonsäure die 7(2-Triphenylmethylamino-thiazol-4-yl)-glyoxylamido-3-[(5-methyl-1,3,4-thiadiazol-2-yl)-thiomethyl] $\Delta$3-cephem-ß-S-oxyd-4-carbonsäure als cremefarbene Festsubstanz mit Rf 0,52 (Fließmittel wie Beispiel 1)
IR in KBr : Lactam CO : 1792 $cm^{-1}$

Beispiel 3:

In der im Beispiel 1 angegebenen Weise erhält man bei der Verwendung von 7((-2-Triphenylmethylamino-thiazol-4-yl)-glyoxylamido)-3- [(1-methyltetrazol-2-yl)-thiomethyl-△3-cephem-4-carbonsäure die 7 [(2-Triphenylmethylamino-thiazol-4-yl)-glyoxylamido] -3- [(1-methyltetrazol-2-yl)-thiomethyl-△3-cephem-ß-S-oxyd-4-carbonsäure als beigefarbenes Festprodukt mit Rf 0,50 (Fließmittel wie in Beispiel 1)
IR in KBr : Lactam CO : 1798 $cm^{-1}$

Beispiel 4:

In der in Beispiel 1 angegebenen Weise erhält man bei der Verwendung von 7((2-Triphenylmethylamino-thiazol-4-yl)-glyoxylamido] -3-methyl-△3-cephem-4-carbonsäure die 7 [(2-Triphenylmethylamino-thiazol-4-yl)-glyoxylamido] -3-methyl-△3-cephem-ß-S-oxyd-4-carbonsäure als gelbbraunen Feststoff mit Rf 0,58 (Fließmittel wie in Beispiel 1)
IR in KBr : 1795 $cm^{-1}$

Beispiel 5:

2,0 g des im Beispiel 1 erhaltenen 7 [2(Triphenylmethylamino-thiazol-4-yl)-glyoxylamido] -cephalosporansäure-ß-S-oxyds werden bei Raumtemp. in einer Mischung aus 8 ml Ameisensäure und 2 ml Wasser eingetragen und 60 Min. gerührt. Nach kurzzeitiger Auflösung beginnt die Abscheidung des Triphenylcarbinols.

Die Reaktionsmischung wird zur Trockne eingeengt, das Triphenylcarbinol mit Äther entfernt, der erhaltene Rückstand isoliert und getrocknet. Man isoliert 7 [(2-Aminothiazol-4-yl)-glyoxylamido] -cephalosporansäure-ß-S-oxyd als orangefarbenen Feststoff, der ohne

zu Schmelzen sich beim Erhitzen oberhalb 240°C dunkel färbt. Rf 0,33 (Fließmittel wie in Beispiel 1) IR in KBr : ß-Lactam 1780 $cm^{-1}$

Beispiel 6:

Aus 2-Amino-thiazol-4-yl-glyoxylsäure wird mit Phosgen und Dimethylacetamid das 2-Aminothiazol-4-yl-glyoxylsäure-chlorid-hydrochlorid hergestellt, das mit einer Lösung von 7-Aminocephalosporansäure in Methylenchlorid, Triäthylamin und Pyrrolidon zum 7[(2-Aminothiazol-4-yl)-glyoxylamido]-cephalosporansäure reagiert. (Cremefarbener Feststoff, Rf 0,38)
1,1 g der so hergestellten 7[(2-Aminothiazol-4-yl)-glyoxylamido]-cephalosporansäure werden bei 0°C in eine Mischung aus 8 ml Eisessig und 2 ml Wasser eingetragen, wobei sich eine Suspension bildet.
Anschließend wird die Lösung unter guter Kühlung mit einer Mischung aus 1 ml 40 % Peressigsäure in Eisessig und 5 ml Eisessig tropfenweise unter Rühren versetzt. Nach 1 Stunde wird von wenigen ungelösten Anteilen abfiltriert, das Lösungsmittel im Vakuum abgezogen und der Rückstand mit Äther verrieben.
Man isoliert das 7[(2-Aminothiazol-4-yl)-glyoxylamido]-cephalosporansäure-ß-S-oxyd als orangefarbenen Feststoff, der nach IR und Dünnschichtchromatogramm mit dem Produkt aus Beispiel 5 identisch ist.

Beispiel 7:

In der in Beispiel 5 beschriebenen Weise erhält man aus der 7((2-Triphenylmethyl-amino-thiazol-4-yl)-glyoxylamido)-3-[(5-methyl-1,3,4-thiadiazol-2-yl)-thiomethyl]-△3-cephem-ß-S-oxyd-4-carbonsäure (Beispiel 2) die 7((2-Aminothiazol-4-yl)-glyoxylamido)-3-[(5-methyl-1,3,4-thiadiazol-2-yl)-thiomethyl]-△3-cephem-ß-S-oxyd-4-carbonsäure als beigefarbenen Feststoff mit Rf 0,32

(Fließmittel wie in Beispiel 1)

Beispiel 8:

In der im Beispiel 5 beschriebenen Weise erhält man aus der 7[(2-Triphenylmethylamino-thiazol-4-yl)-glyoxylamido)-3-[(1-methyltetrazol-2-yl)-thiomethyl]-△3-cephem-ß-S-oxyd-4-carbonsäure (Beispiel 3) die 7[(2-Aminothiazol-4-yl)-glyoxylamido)-3-[(1-methyltetrazol-2-yl)thiomethyl]-△3-cephem-ß-S-oxyd-4-carbonsäure als orangefarbenen Feststoff mit Rf 0,30 (Fließmittel wie Beispiel 1)

Herstellung der Ausgangsverbindungen

a) Bromacetglyoxylsäureäthylester

120 g Acetglyoxylsäureäthylester werden in 700 ml Methylen-chlorid suspendiert und bei 5°C zunächst mit 10 ml einer 40 % HBr in Eisessiglösung versetzt und anschließend innerhalb von 1 Stunde mit einer Lösung von 146 g Brom in 200 ml Methylenchlorid umgesetzt. Nach dem Entfärben der Lösung wurde das Lösungsmittel abgezogen und das zurückbleibende Öl ohne weitere Reinigung umgesetzt.

b) 2-Amino-thiazol-4-yl-glyoxylsäureäthylester

Eine Lösung von 66 g Thioharnstoff in 450 ml Öthanol werden bei 5°C tropfenweise mit 195 g Bromacetglyoxylsäureäthylester versetzt. Nach Ende der Zugabe wird 30 Min. bei Zimmertemperatur und 30 Min. bei 50°C gerührt und anschließend die erhaltene Reaktionsmischung nach Versetzen mit Aktivkohle filtriert.
Das Filtrat wird durch Zugabe von Natriumbicarbonatlösung auf pH 7 gestellt, wobei der 2-Aminothiazol-4-yl-glyoxylsäureäthylester in Kristallen von Schmp. 147°C

auskristallisiert.
Falls gewünscht kann der Ester in an und für sich bekannter Weise in die freie Säure überführt werden.

c) 2-Triphenylmethylamino-thiazol-4-yl-glyoxylsäureäthylester

Eine Lösung von 90 g 2-Aminothiazol-4-yl-glyoxylsäureäthylester in 225 ml Dimethylformamid und 375 ml $CH_2Cl_2$ wird bei -15°C mit 27 g Triäthylamin und anschließend bei -30°C mit 75 g Triphenylchlormethan versetzt. Nach 15 Min. bei -30°C wird 3 Stunden ohne Kältebad gerührt, die erhaltene Reaktionsmischung mit 500 ml $CH_2Cl_2$ versetzt, mit 300 ml 1 n HCl und anschließend zweimal mit 200 ml Wasser gewaschen, die organische Phase über $Na_2SO_4$ getrocknet und das Lösungsmittel abgedampft. Es bleibt ein Öl, das für die weiteren Umsetzungen ohne vorherige Reinigung verwendet wurde.

d) 2-Triphenylmethylamino-thiazol-4-yl-glyoxylsäure

Eine Lösung von 156 g rohem 2-Triphenylmethylamino-thiazol-4-yl-glyoxylsäureäthylester in 150 ml Methanol wird mit einer Lösung von 14,8 g NaOH in 370 ml Methanol versetzt, 5 Minuten unter Rückfluß gekocht, wobei das Natriumsalz der 2-Triphenyl-methylaminothiazol-4-yl-glyoxylsäure auskristallisiert.
Das erhaltene Natriumsalz wird in 380 ml Wasser suspendiert und unter heftigem Rühren mit 76 ml 2 n HCl versetzt. Nach 15 Min. wird der Niederschlag abgesaugt, mit Wasser gewaschen und getrocknet.
Man erhält die 2-Triphenylmethylamino-thiazol-4-yl-glyoxylsäure als gelbe Kristalle vom Schmp. 163 - 165°C (Zers.).

e) 7[(2-Triphenylmethylamino-thiazol-4-yl)-glyoxylamido]-cephalosporansäure

Eine Suspension von 9,1 g (2-Triphenylmethylamino-thiazol-4-yl)-glyoxylsäure in 50 ml Methylenchlorid wird mit 2,4 g Triäthylamin versetzt, die erhaltene Lösung unter Stickstoff und Feuchtigkeitsausschluß auf -50°C gekühlt und mit einer Lösung von 2,66 g Pivaloylsäurechlorid in 200 ml Methylenchlorid versetzt. Nach Ende der Zugabe wird 2 Stunden bei 0°C nachgerührt, anschließend die Lösung des gemischten Anhydrids erneut auf -50°C gekühlt und mit einer Lösung von 5,44 g 7-Aminocephalosporansäure in einer Mischung aus 50 ml Methylenchlorid und 4,04 g Triäthylamin versetzt. Nach Ende der Zugabe wird ohne Kältebad 3 Stunden nachgerührt, anschließend das Lösungsmittel im Vakuum abgezogen, der Rückstand in 500 ml Wasser aufgenommen, mit 2 n HCl auf pH 1 angesäuert und sofort mehrfach mit Methylenchlorid aufgenommen. Nach dem Trocknen wird die organische Phase entfernt und der Rückstand mit Äther verrieben. Man isoliert die 7-((2-Triphenylmethylamino-thiazol-4-yl)-glyoxylamido)-cephalosporansäure als Festprodukt vom Schmp. 155 - 158°C Zers..
Rf (Butanol, $H_2O$, Äthylalkohol, Essigsäure 5 : 2 : 1,5 : 1,5) 0,63

f) 7[(2-Triphenylmethylamino-thiazol-4-yl)-glyoxylamido]-3[(5-methyl-1,3,4-thiadiazol-2-yl)-thiomethyl]-3-cephem-4-carbonsäure

In der im Beispiel e angegebenen Weise erhält man bei Verwendung von 7-Amino-3((5-methyl-1,3,4-thiadiazol-2-yl)-thiomethyl)-3-cephem-4-carbonsäure, 7((2-Triphenylmethylamino-thiazol-4-yl)-glyoxylamido)-3[(5-methyl-1,3,4-thiadiazol-2-yl)-thiomethyl]-3-cephem-4-carbonsäure als Feststoff vom Schmp. 135 - 140°C, Rf (Fließmittel wie in Beispiel 1) 0,58

g) 7[(2-Triphenylmethylamino-thiazol-4-yl)-glyoxyl-amido]-3[(1-methyl-tetrazol-2-yl)-thiomethyl]-3-cephem-4-carbonsäure

In der im Beispiel e angegebenen Weise erhält man bei Verwendung von 7-Amino-3((1-methyl-tetrazol-2-yl)-thiomethyl)-3-cephem-4-carbonsäure die 7[(2-Triphenyl-methylamino-thiazol-4-yl)-glyoxylamido]-3[(1-methyl-tetrazol-2-yl)-thiomethyl]-3-cephem-4-carbonsäure als Feststoff vom Schmp. 145 - 148°C.
Rf (Fließmittel wie in Beispiel 1) 0,56

Patentansprüche

1. Cephemderivate der allgemeinen Formel I

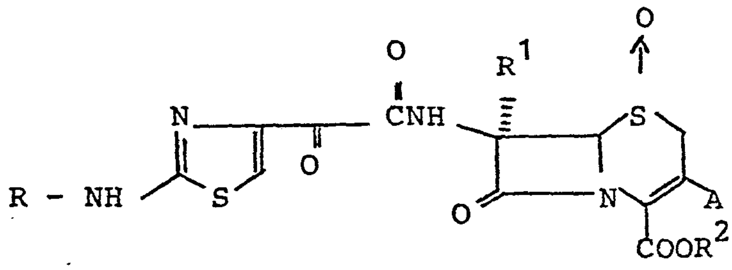

I

in der R Wasserstoff oder eine Aminoschutzgruppe, $R^1$ Wasserstoff oder niedrigmolekulares Alkoxy, $R^2$ Wasserstoff oder ein pharmazeutisch unbedenkliches Kation und A eine Methylgruppe, eine Gruppe $-CH_2O$-Acyl mit niedrigmolekularem Acyl oder einen Rest $-CH_2-SR^3$ bedeutet, in dem $R^3$ für einen gegebenenfalls substituierten 5- oder 6-gliedrigen heterocyclischen Ring mit 1 bis 4 Heteroatomen steht.

2. Verfahren zur Herstellung von Cephemderivaten der allgemeinen Formel I, dadurch gekennzeichnet, daß man Cephemverbindungen der allgemeinen Formel II

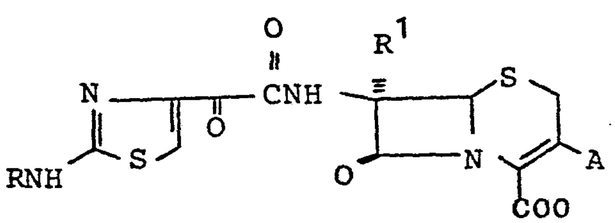

II

in der die Reste A, R, $R^1$ und $R^2$ die oben angegebenen Bedeutungen haben, oxydiert oder

ein 7-Aminocephalosporansäure-S-oxyd der allgemeinen Formel III

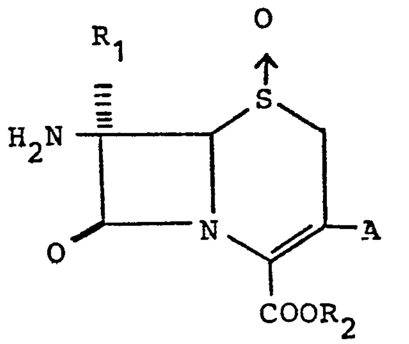

III

in der A, $R_1$ und $R_2$ die obengenannten Bedeutungen besitzen, mit zur Amidbildung befähigten Derivaten einer Säure der allgemeinen Formel IV

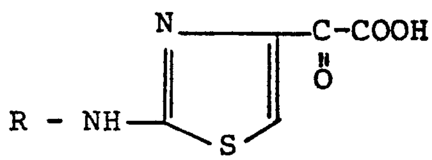

IV

in der R die vorstehende Bedeutung besitzt, umsetzt und, falls gewünscht, in den Verbindungen der Formel I den Rest R in der Bedeutung einer Aminoschutzgruppe abspaltet.

3. Gegen bakterielle Infektionen wirksame pharmazeutische Präparate, gekennzeichnet durch einen Gehalt an Cephemderivaten der allgemeinen Formel I.

4. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

5. Verwendung von Cephemderivaten der allgemeinen Formel I zur Bekämpfung bakterieller Infektionen.

0005785

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 79 10 1549

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 223 375 (GLAXO)<br>* Seiten 121-147; Ansprüche 2,3,6, 7,16,18,98-105 *<br>-- | 1-5 |
| | DE - A - 2 710 282 (FUJISAWA)<br>* Seiten 1-21; Ansprüche 1,43,50, 51 *<br>-- | 1-5 |
| P | DE - A - 2 716 677 (HOECHST)<br>* Seiten 1-4; Ansprüche 1-3,5 *<br>-- | 1-5 |
| P | DE - A - 2 710 902 (HOECHST)<br>* Seiten 1-4; Ansprüche 1,4-6 *<br>---- | 1-5 |

KLASSIFIKATION DER ANMELDUNG (Int.Cl.³)

C 07 D 501/00
A 61 K 31/545//
C 07 D 277/40
277/42

RECHERCHIERTE SACHGEBIETE (Int. Cl.³)

C 07 D 501/00
A 61 K 31/545

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:
Unvollständig recherchierte Patentansprüche:
Nicht recherchierte Patentansprüche: 5
Grund für die Beschränkung der Recherche: Verfahren zur chirurgischen oder therapeutischen Behandlung des menschlichen oder tierischen Körpers. (Siehe Art. 52(4) des Europäischen Patentübereinkommens)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 23-08-1979 | LUYTEN |